# EUROPEAN PATENT APPLICATION

(11) **EP 4 480 429 A1**
(43) Date of publication of application: **25.12.2024**
(21) Application number: 23180410.5
(22) Date of filing: 20.06.2023
(51) Int. Cl.: A61B 17/80, A61F 2/28, A61F 2/30, B33Y 80/00

(54) **IMPLANT AND METHOD FOR COVERING LARGE-SCALE BONE DEFECTS FOR THORAX**

(30) Priority: 19.06.2023 US 202318337284
(71) Applicant: Karl Leibinger Asset Management GmbH & Co. KG, 78570 Mühlheim an der Donau (DE)
(72) Inventor: AKSU, Adem, 78054 VS-Schwenningen (DE); KLEIN, Vanessa, 78532 Tuttlingen (DE); REINAUER, Frank, 78576 Emmingen-Liptingen (DE)
(74) Representative: Isarpatent

(57) **Abstract**

The invention provides an implant (100-1200) for covering a thorax bone defect(3), comprising:
at least one lattice structure (140-1240) of individual lattice cells (110-810), each lattice cell (110-810) comprising one or more ring elements (120), each ring element (120) comprising a through-hole configured for receiving a fixation device (11), at least some of the ring elements (120) of each lattice structure (140-1240) being connected via linear or non-linear bridge elements (130, 431, 432, 531, 532, 731, 732) to one another, wherein the implant (100-1200) is made at least predominantly from a plastic material. The invention also provides a system comprising such an implant and a plurality of fixation devices, as well as a method for manufacturing such an implant.

## Description

### FIELD OF THE INVENTION

The present invention relates to an implant and methods for covering large-scale bone defects, particularly in the thorax. Covering a bone defect in particular includes bridging the bone defect, i.e. providing a connection or coupling between bone portions remaining in a patient's body.

### BACKGROUND OF THE INVENTION

For the covering of large-scale bone defects in the thorax area, usually so-called rib plates are employed. Rib plates are usually strips of resilient material comprising through-holes (or: orifices) for receiving fixation devices such as bone screws. Rib plates may be provided in stiff, semi-stiff, or malleable variants and are usually fixed to remaining portions of the ribs of a patient, bridging gaps in between. In some cases, however, additional support for the areas between different ribs would be even more advantageous for the treatment.

For the treatment of skull defects, metallic mesh grid implants are known, for example from US 5468242 A. Such mesh grid implant offer the flexibility needed to conform to curved portions of the skull while still providing sufficient resilience.

In the thorax area, flexibility to adapt to various curvatures is also important, while the constant movement of the thorax due to the breathing or movement of the patient provides additional challenges. At the same time, the internal organs need to be protected from harm, in particular important organs such as the heart, the lungs, and the main blood vessels which are normally protected by the thorax.

Thoracic trauma is an injury of the bones of the chest and/or of organs or organ systems therein, and which is often life-threatening. One of the most common injuries of the thorax is a rib fracture. Rib fractures can commonly be treated using metallic implants and bone reconstruction. Known is also the use of so-called K-wires ("Kirschner wires") in K-wire osteosynthesis. However, these can be prone to movement after implantation, in particular in bone cavities, and may break. Also, when using K-wires, often an additional plaster cast is necessary, which may complicate the treatment of thoracic trauma.

Also known is the use of metal rails, or the use of rib plates. For example, DE 38 03 435 C1 describes a rail implant with a side surface adapted to the contour of a patient's rib. This solution is improvable with respect to its stiffness, which may impact breathing.

### SUMMARY OF THE INVENTION

It is one of the objects of the present invention to provide enhanced implants, in particular for covering large-scale bone defects in the thorax. Additional objects are that the implant has a high threshold for material failure, does not cause any pain, aids in healing, and allows controlled movement in the region of the defect.

Further objects are the providing of improved methods for manufacturing such implants, improved methods for covering large-scale bone defects, and other improved products related thereto.

The above mentioned objects are solved by the subject-matter of the independent claims.

Thus, according to a first aspect of the present invention, an implant for covering a thorax bone defect is provided, the implant comprising:
at least one lattice structure of individual lattice cells, each lattice cell comprising one or more ring elements, each ring element comprising a through-hole configured for (or: capable of) receiving a fixation device,
at least some of the ring elements of each lattice structure being connected via linear or non-linear bridge elements to one another,
wherein the implant is made at least predominantly from a plastic material.

This provides an implant, preferably made from one or more polymers, for treating a small or large-scale thorax bone defect. The implant is, due to the ring elements and its through-holes, easily fixed to any bone (e.g., a rib) in a large number of different orientations and positions according to the decision of a surgeon. Moreover, the implant may be bendable around one or more torsion axis and/or bending axis.The implant is in particular suitable for large-scale bone defects of the thorax. Large-scale bone defects are defined herein as bone defects with an area larger than 1 square millimeters, in particular larger than 1000 square millimeters, for example larger than 25 square centimeters, 100 square centimeters or even 900 square centimeters.

The implant is also well suited for treating curved bone defects as well as bone defects of irregular shape, in particular since it can be adapted by a surgeon even during surgery to the specific size and shape of the defect, for example by removing (e.g., cutting) excess portions of the implant.

The bridge elements advantageously have a cross-sectional area of less than 20mm, which allows them to be easily cut through using common surgical instruments.

Each lattice structure may be two-dimensional implementation of a triclinic lattice, a monoclinic lattice, an orthorhombic lattice, a tetragonal lattice, a hexagonal lattice or a cubic lattice.

The implant may comprise more than 10, more than 50, or more than 100 ring elements and a corresponding number of through-holes, giving the surgeon a large number of choices of where to insert a fixation device and at which points to fix the implant with a portion of bone of the patient.

According to a second aspect, the invention provides a system for covering a large-scale bone defect, comprising the implant according to any embodiment of the first aspect, as well as a plurality of fixation devices.

The fixation devices may be identical or different from one another. They may comprise, or consist of, the same material or materials as the implant, or different material(s). Exemplary fixation devices may be comparatively simple bone screws, but also more complex devices such as ones that may be fixed by ultrasound welding.According to a third aspect, the invention provides a method for covering a thorax bone defect, in particular a large-scale thorax bone defect, the method comprising at least steps of: providing the implant according to any embodiment of the first aspect and at least two fixation devices, or a system according to any embodiment of the second aspect of the invention; and
fixing the implant to at least two portions of bone of a patient using the fixation devices.

According to a fourth aspect, the invention provides a method for producing the implant according to any embodiment of the first aspect of the invention, the method comprising, comprising additive manufacturing, AM, or injection molding of the entire implant. Producing the implant may comprise first casting the implant, and then reshaping it into a desired shape, for example to produce curved structures or elements.

Producing the implant by injection molding may comprise:
- (optionally) pre-shaping an implant body mold;
- injecting a plastic, preferably a polymer, material into the implant body mold; and
- allowing the injected material to cool and cross-link.

According to a fifth aspect, the invention provides a non-volatile, computer-readable data storage medium storing data which defines a digital representation of the implant according to any of the embodiments of the first aspect of the present invention.

According to a sixth aspect, the invention provides a non-volatile, computer-readable data storage medium storing data which defines a digital representation of the implant according to any of the embodiments of the first aspect of the present invention. The data storage medium may be a solid state memory, a magnetic hard drive, a solid state drive, a CD, a DVD, a Blu-Ray and/or the like.

According to a seventh aspect, the invention provides a data stream comprising, or configured to generate, data defining a digital representation of the implant according to any of the embodiments of the first aspect of the present invention.

The data in any of the embodiments according to the fifth, sixth, or seventh aspect may further define operating instructions adapted to control an additive manufacturing device to fabricate the implant using the digital representation of the implant when said data is relayed to the additive manufacturing device.

Further advantageous embodiments, variants, options, and refinements of embodiments are evident from the dependent claims as well as from the specification and the accompanying drawings.In some advantageous embodiments, variants, or refinements of embodiments, the implant is made from a plastic material using additive manufacturing, AM, and/or injection molding. This provides a comprehensive and easy way of producing an implant with desired properties of sturdiness, flexibility, and the like.

In some advantageous embodiments, variants, or refinements of embodiments, the implant is made from a material comprising, or consisting of: polyetheretherketone, PEEK, Polyethylen, PE, polyphenylsulfone, PPSU, polyetherketoneketone, PEKK, Magnesium, Molybdenum and/or a compound therewith.

In some advantageous embodiments, variants, or refinements of embodiments, the implant is made from a material comprising, or consisting of: a compound including hydroxylapatite, HA, tricalciumphosphate, TCP, Silver, and/or Magnesium. It has been found that these materials offer the desired balance between weight, sturdiness, flexibility, and medical properties.

In some advantageous embodiments, variants, or refinements of embodiments, at least the at least one lattice structure has anti-bacterial properties. This supports the healing process. Other parts of the implant, such as bar units (see below) or the like may also be provided with anti-bacterial properties. Preferably, the entire implant has anti-bacterial properties.

In some advantageous embodiments, variants, or refinements of embodiments, the at least one lattice structure has a thickness of from about 0.1 mm to about 5 mm.

In some advantageous embodiments, variants, or refinements of embodiments, the lattice structure is an essentially 2-dimensional lattice, in particular a triclinic lattice, a monoclinic lattice, an orthorhombic lattice, a tetragonal lattice, a hexagonal lattice or a cubic lattice.

In some advantageous embodiments, variants, or refinements of embodiments, at least some lattice cells consist of a single ring element, together with at least a portion of at least one bridge element.

In some advantageous embodiments, variants, or refinements of embodiments, all connections (in particular: all bridge elements) between all ring elements are non-linear. Non-linear connections, in particular bridge elements, provide additional flexibility.

In some advantageous embodiments, variants, or refinements of embodiments, the implant comprises at least one lower-density lattice cell, LDLC, and at least one higher-density lattice cell, HDLC, having the same size and contour as the at least one lower-density lattice cell, LDLC, but comprising a higher number of ring elements. In this way, properties of different portions of the implant may be made different from one another, e.g., higher-density lattice cell portions may be more robust and provide stiffer support, while lower-density lattice cell portions may be more flexible.

In some advantageous embodiments, variants, or refinements of embodiments, the implant comprises at least one lattice structure and at least one bar unit, the lattice structures and the bar units being arranged in a regular pattern. The lattice structures and the bar units may be arranged alternatingly. The lattice cells immediately adjacent to bar units may be connected to the bar units by linear bridge elements (although also non-linear bridge elements are possible).

In some advantageous embodiments, variants, or refinements of embodiments, the bridge elements connecting ring elements in a direction in parallel to the longitudinal direction of the at least one bar unit are linear bridge elements. In this way, additional support is provided in the longitudinal direction, while maintaining more flexibility in a direction perpendicular to it.

In some advantageous embodiments, variants, or refinements of embodiments, the implant comprises a plurality of bar units arranged in a staggered manner with respect to one another.

In some advantageous embodiments, variants, or refinements of embodiments, the implant comprises a plurality of bar units arranged in parallel with one another, and exactly aligned with respect to their longitudinal ends.

In some advantageous embodiments, variants, or refinements of embodiments, each bar unit comprise at least one line of through-holes for receiving fixation devices. The at least one bar unit, or preferably each bard unit, may comprise a plurality of pairs of through-holes, the pairs being arranged along the longitudinal axis of the bar unit. The pairs of the plurality of pairs of through-holes may preferably be arranged in parallel to each other but neither in parallel nor perpendicular to the longitudinal axis of the at least one bar unit.

In some advantageous embodiments, variants, or refinements of embodiments of the system according to the second aspect of the present invention, the fixation devices comprise, or consist of, bio-degradable or non-bio-degradable metal screws or pins, or resorbable or non-resorbable polymeric pins or screws.

In some advantageous embodiments, variants, or refinements of embodiments of the method according to the third aspect of the present invention, the at least two portions of bone are portions of one or more ribs, wherein in particular one or more ribs have been partially resected.

In some advantageous embodiments, variants, or refinements of embodiments of the method according to the third aspect of the present invention, the system comprises an implant according with at least one bar unit, wherein the implant is fixed, by one or more ring elements of at least one lattice structure or of another bar unit, to at least one rib of a patient, such that the at least one bar unit substitutes at least a missing section of another rib of the patient.

In some advantageous embodiments, variants, or refinements of embodiments of the method according to the third aspect of the present invention, the implant, after manufacturing, is tailored, particularly by removing parts of at least one lattice structure, to a specific patient and/or a specific bone defect.

### BRIEF DESCRIPTION OF THE DRAWINGS

The invention will be explained in greater detail with reference to exemplary embodiments depicted in the drawings as appended.

The accompanying drawings are included to provide a further understanding of the present invention and are incorporated in and constitute a part of this specification. The drawings illustrate the embodiments of the present invention and together with the description serve to explain the principles of the invention. Other embodiments of the present invention and many of the intended advantages of the present invention will be readily appreciated as they become better understood by reference to the following detailed description. The elements of the drawings are not necessarily to scale relative to each other. Like reference numerals designate corresponding similar parts.
- Fig. 1A: shows an implant for covering a large-scale bone defect according to an embodiment of the present invention;
- Fig. 1B: shows a variant of the embodiment of Fig. 1A;
- Fig. 1C: shows a variant of the embodiment of Fig. 1B;
- Fig. 2: illustrates an implant according to another embodiment of the present invention;
- Fig. 3: illustrates an implant according to yet another embodiment of the present invention;
- Fig. 4: illustrates an implant according to still another embodiment of the present invention;
- Fig. 5: illustrates an implant according to another embodiment of the present invention;
- Fig. 6: illustrates an implant according to yet another embodiment of the present invention;
- Fig. 7: illustrates an implant according to still another embodiment of the present invention;
- Fig. 8: illustrates an implant according to another embodiment of the present invention;
- Fig. 9: illustrates an implant according to yet another embodiment of the present invention;
- Fig. 10: illustrates an implant according to still another embodiment of the present invention;
- Fig. 11: illustrates an implant according to another embodiment of the present invention;
- Fig. 12: illustrates an implant according to yet another embodiment of the present invention;
- Fig. 13: illustrates a possible utilization of the implant of Fig. 8;
- Fig. 14: illustrates a possible utilization of the implant of Fig. 1A;
- Fig. 15: schematically illustrates a system comprising an implant and a fixation device;
- Fig. 16: shows a schematic flow diagram illustrating a method for covering a bone defect according to an embodiment;
- Fig. 17: shows a schematic flow diagram illustrating a method for manufacturing an implant according to an embodiment; and
- Fig. 18: shows a schematic block diagram illustrating a data storage medium according to an embodiment of the present invention.

In the figures, like reference numerals denote like or functionally like components, unless indicated otherwise. Any directional terminology like "top", "bottom", "left", "right", "above", "below", "horizontal", "vertical", "back", "front", "row", "column", and similar terms are merely used for explanatory purposes and are not intended to delimit the embodiments to the specific arrangements as shown in the drawings.

### DETAILED DESCRIPTION OF THE INVENTION

Although specific embodiments have been illustrated and described herein, it will be appreciated by those of ordinary skill in the art that a variety of alternate and/or equivalent implementations may be substituted for the specific embodiments shown and described without departing from the scope of the present invention. Generally, this application is intended to cover any adaptations or variations of the specific embodiments discussed herein.

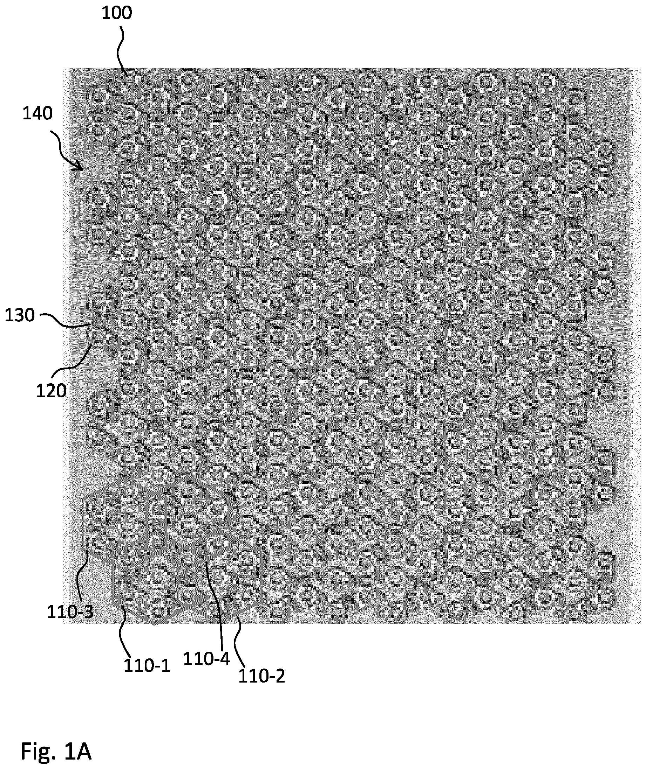

Fig. 1A shows an implant 100 for covering a large-scale bone defect according to an embodiment of the present invention. In this embodiment, the implant 100 consists of a single lattice structure 140 of individual cells 110-1, 110-2, 110-3, 110-4 (in the following sometimes collectively designated as 110-i). The lattice structure 140 here has a hexagonal primitive structure in two dimensions: each lattice cell 110-i has the form of a hexagon, wherein each corner of the hexagon is formed by a ring element 120. It shall be understood that, as has been described in the foregoing, any lattice structure described herein may be formed with any other type of periodic lattice pattern as well, in particular with a triclinic, monoclinic, orthorhombic, tetragonal, trigonal, hexagonal or cubic lattice pattern.

Each ring element 120 comprises a through-hole (or: orifice) for receiving a fixation device, such as a bone screw, a pin, or the like. For example, the size of a through-hole, or the diameter of a ring element 120, may be between 0.1mm to 5.5mm.

A "ring element" in this context must not necessarily have a circular contour, i.e., not the entire ring element 120 must be ring-shaped. Also possible are square-shaped or oblong ring elements 120, with a circular through-hole. However, ring-shaped ring elements 120 are preferred as they interfere the least with the tissue they may be covering.

The ring elements 120 are connected to one another by non-linear bridge elements 130, which generally have an S-shape, wherein the curvature of the bridge element 130 changes direction at least once, preferable at the halfway point as an inflection point.

As is evident in Fig. 1A, lattice cells 110-1, 110-2, 110-3 on the outer edge of the lattice structure 140 are slightly different from lattice cells 110-4 that are completely inside the lattice structure 140 regarding the bridge elements 130, which are missing on the outer edge of the lattice structure 140. Aside from this difference, all of the lattice cells 110-i are identical so that the lattice structure 140 can be designated as a hexagonal-primitive lattice (similar to how crystals are described, which also have a slightly different configuration at their outer edges).

Fig. 1A also shows that some parts of the lattice structure 140 can belong to multiple lattice cells 110-i, for example the ring element 120 belonging to each of the lattice cells 110-1, 110-2 and 110-4.

In the configuration of the implant 100 of Fig. 1A, each lattice cell 110-i (or: unit cell, or: lattice unit cell) has a hexagonal contour, wherein the lattice cells 110-i are defined such that the ring elements 120 of their hexagonal contour are directly connected, via the non-linear bridge elements 130, to each of the two adjacent ring elements 120 along the hexagonal contour. Each lattice cell 110-i further comprises, apart from the six elements of its hexagonal contour, a central ring element 120 arranged at the center of the hexagonal contour. This central ring element 120 is, in the shown configuration, directly connected to only two of the six other ring elements 120, which are arranged diagonally opposite one another.

It shall be understood that the specific configuration shown in Fig. 1A has been found to be especially advantageous, as it yields a comparatively stiff yet still adaptive lattice structure 140. The stiffness stems in part from the central ring elements 120 which, in some variants, can also be left out.

As has been described in the foregoing, the entire lattice structure 140, or the entire implant 100, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

The configuration in Fig. 1A is such that the overall lattice structure 140 has a generally rectangular shape, with deviations of only up to half a width of a lattice cell 110-i.

The implant 100 (and more specifically, the lattice structure 140 thereof) may have a thickness of between 0,1 mm and 5 mm, preferably between 1mm and 3mm, and may be provided with anti-bacterial properties. One or both of the planar surfaces of the implant, in particular the lattice structure 140, may have a rough texture so as to improve the integration of living cells thereto or therein.

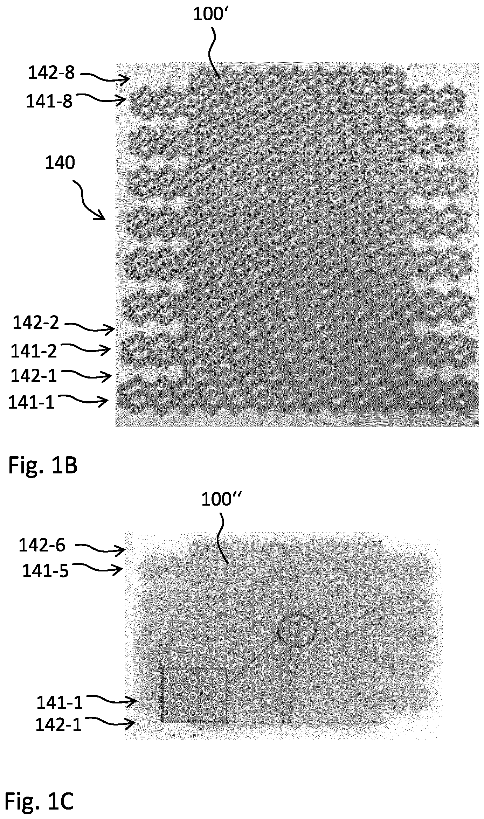

Fig. 1B shows a variant implant 100' of the implant 100 which consists of a lattice structure 140' with the same lattice regularity and the same individual lattice cells 110-i as the lattice structure 140', but a different overall shape. In the variant of Fig. 1B, the lattice structure 140' is arranged with alternating long strip sections 141-1, 141-2, ...,141-8 and short strip sections 142-1, 142-2,...,142-8, the long strip sections 141-i being longer than the short strip sections 142-i.

Short and long strip sections are each one lattice cell 110-I wide and are arranged alternatingly, adjacent and in parallel, starting with a long strip section 141-1 and ending with a short strip section 142-8. The long strip sections 141-i are arranged symmetrically about the short strip section 142-i, in the shown variant such that on each side the long strip sections 141-i extend for 2.5 lattice cells 110-i longer as compared to the short strip sections 142-i.

For example, the long strip sections 141-i may be used for fixing to rib sections of a patient on one or both sides, much as a rib plate in the prior art.

Fig. 1C show another variant implant 100" of the implant 100'. The implant 100" has the same lattice regularity and the same individual lattice cells 110-i as the lattice structures 140, 140', but again a different overall shape: The lattice structure 140" is arranged with five long strip sections 141-1, ..., 141-5, parallel and alternating with six short strip sections 141-2,142-6, starting and ending with short strip sections 142-1, 142-6, wherein long and short strip sections are otherwise configured as has been described in the foregoing with respect to Fig. 1B.

Because of the above-mentioned selection of materials, a physician may - literally - tailor the implant 100, 100', 100" to a patient, for example starting with the implant 100' of Fig. 1B, and using scissors or a knife to sever three long strip sections 141-1, 141-2, 141-3 and two short strip sections 142-1, 142-2 in order to obtain the implant 100", even during a medical procedure.

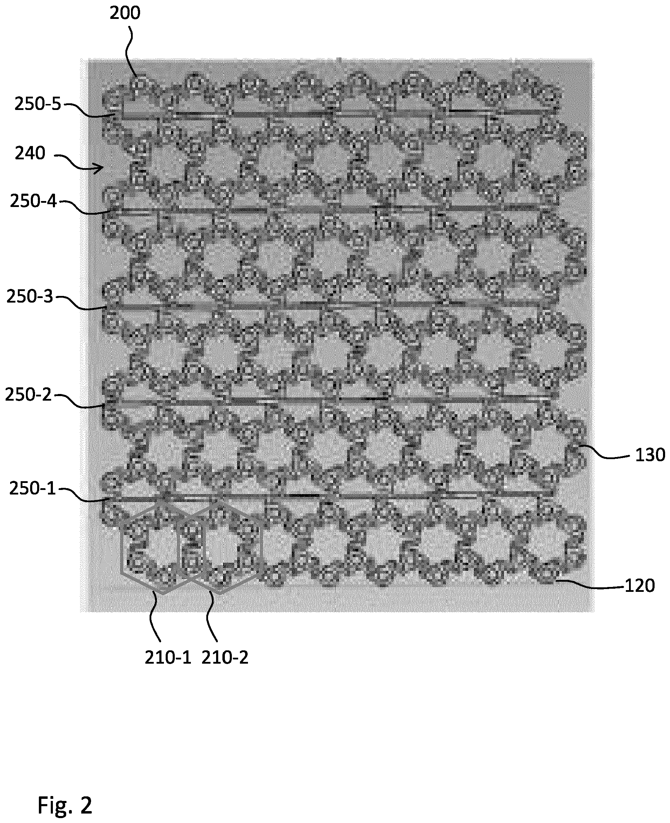

Fig. 2 illustrates an implant 200 according to another embodiment of the present invention. The implant 200 comprises a lattice structure 240 of hexagonal-primitively arranged hexagonal lattice cells 210-1, 210-2, ..., 210-i. In contrast to the lattice cells 110-i of Fig. 1A-1C, the lattice cells 210-i only consist of the six ring elements 120 forming the hexagonal contour, and do not contain any center ring elements. In general, the lattice structure 240 of Fig. 2 is therefore less stiff than the one of Figs. 1A-1C.

However, the implant 200 comprises a number of bar units 250-1, 250-2, ..., 250-5 which are arranged in regular intervals in parallel, crossing the (empty) center of the lattice cells 210-i of each second row of lattice cells 210-i. The bar units 250-i add additional resilience, especially against stretching in the longitudinal direction of the bar units 250-I, while offering some freedom of movement for, e.g., shearing in other directions. The bar units 250-i may, for example, have a thickness between 0.5 mm and 5mm. Optionally, the have a larger thickness than the lattice structure(s) 240, thus giving additional stability to the implant 200.

The entire implant 200, or any parts thereof such as the bar units 250-i and/or the lattice structure(s) 240, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

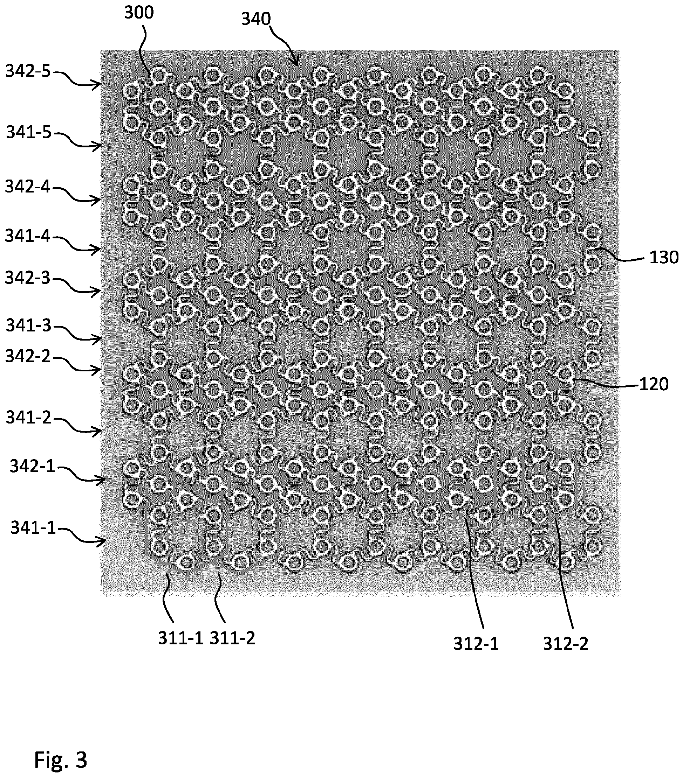

Fig. 3 illustrates an implant 300 according to another embodiment of the present invention. The implant 300 comprises a lattice structure 340 of hexagonal-primitively arranged hexagonal lattice cells of two varieties: a first type of strip section 341-1, 341-2,...341-5 and a second type of strip section 342-1, 342-2,...,342-5 are arranged in parallel and alternatingly.

The overall structure is the same as of implant 100 in Fig. 1A, only that the first type of strip section 341-1 consists of lattice cells 311-1, 311-2,... of the type of the implant 200 of Fig. 2 (i.e. identical to the lattice cells 210-i), and the second type of strip section 341-2 consists of lattice cells 312-1, 312-2,... of the type of the implant 100 of Fig. 1A (i.e. identical to the lattice cells 110-i). By comparison, the lattice cells 312-i may be designated as higher-density lattice cells, HDLC, and the lattice cells 311-i may be designated as lower-density lattice cells, LDLC, since the HDLCs 312-i have the same size and contour (shape of outline) as the LDLCs 311-i but comprise a higher number (here: one more) of ring elements 120. Thus, the density of through-holes per area and/or the density of material per area (here: both) is higher for the HDLCs 312-i than for the LDLCs 311-i.

As in the foregoing, the implant 300 is shown as starting on one end with a strip section 341-1 of the first type, and ends with a strip section 342-5 of the second type (or vice versa, depending on where beginning and end are defined), but similarly strip sections of either type may be arranged both at the end and at the beginning.

The lattice cells 311-i of the first type of strip section 341-i (or: lattice cells 311-i of the first type) are more flexible than the lattice cells 312-i of the second type of strip section 342-i (or: lattice cells 312-i of the second type). The implant 300 thus offers an advantageous balance of flexibility and robustness. The ratio of first and second types of strip sections may be amended according to the particular needs of a bone defect. For example, there may be one strip section 342-i of the second type (or: stiff type) after every strip section 341-i of the first type (or: flexible type), after every second, after every third, and so on. Similarly, there may be two strip sections 342-i of the second type (or: stiff type) after every one strip section 341-i of the first type (or: flexible type), after every two strip sections 341-i of the first type, after every three, and so on.

The implant 300 of Fig. 3 may be combined with the variants as described with respect to any of the preceding Figs., in particular Fig. 1B and Fig. 1C. Thus, either the strip sections of the first type 341-i or the strip sections of the second type 342-i may be configured as long strip sections and short strip sections, respectively, or vice versa. Preferably, the strip sections of the second type 342-i, which are more resilient, are configured as long strip sections, thus fulfilling some of the functions of conventional rib plates, while the more flexible strip section of the first type 341-i are arranged in between as short strip sections.

The entire implant 300, or any parts thereof such as strip sections 341-i, 342-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

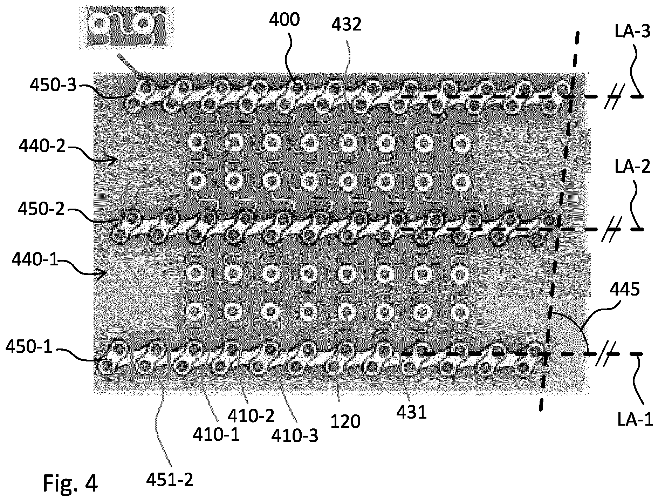

Fig. 4 illustrates an implant 400 according to yet another embodiment of the present invention. The implant 400 comprises two separate lattice structures 440-1, 440-2, each of which is sandwiched by a bar unit 450-1, 450-2, 450-3 on each side.

The bar units 450-i are arranged in parallel to one another, i.e., their respective longitudinal axes LA-1, LA-2, LA-3 are arranged in parallel. In the implant 400, they are furthermore arranged in a staggered configuration with regard to their longitudinal direction, as is indicated by the angle 445 in Fig. 4. The angle 445 is set between any of the longitudinal axes LA-i and a line connecting the outermost points of each of the bar units 450-i on one side. The angle 445 may in some variants be 90° (ninety degrees), although in the embodiment shown in Fig. 400 it is smaller than 90° and larger than 45°, in particular between 60° and 90°, preferably between 75° and 90°.

The bar units 450-i are formed preferably as rib plates according to any known prior art. As such, they may be rigid, flexible, or anything in between. One important application for them is to be fixed along still existing ribs of the patient. In the implant 400, each bar unit 450-i comprises pairs 451-2 of through-holes, the pairs 451-i being arranged in parallel with each other and at an angle to the longitudinal axis LA-i of the bar unit 450-i, said angle being between 30° and 90°, preferably 45°. The through-holes in the bar units 450-i may be of the same size as those in the ring elements 120 such that a surgeon need only prepare one type of fixing device for fixing any of the through-holes of the implant 400.

The lattice structures 440-1, 440-2 of the implant 400 are preferably identically configured, although in variants they could be differently configured, e.g., regarding the number of rows of lattice cells 410-i. Rows in this context should be understood to be lines of lattice cells 410-i that are arranged in parallel to the longitudinal axes LA-i of the bar units 450-i.

Similarly, in the implant 400, each lattice structure 440-1, 440-2 consists of two rows of eight lattice units 410-i each, although other numbers of rows and of lattice units 410-i per row can easily be provided. In the implant 400, each lattice unit 410-i consists of a single ring element 120 and, depending on whether it is arranged on the edge of the lattice structure 440-i or inside of it, parts of three or four bridge elements 130, respectively. The bridge element 130 all have the S-shape that has been previously described. In particular, each bridge element 130 may be formed with two opposing curvatures when moving along the bridge element 130 from one ring element 120 to another.

The lattice regularity of the lattice structures 440-i may be designated as cubic-bodycentered in two dimensions. Each ring element 120 is connected, via one of the S-shaped bridge elements 431, to each of its four nearest neighbours (or three if the ring element 120 in question is on an edge of the lattice structure 440-i).

The bridge elements 432 which connect one of the lattice cells 410-i to one of the bar units 450-i may be the same as the bridge elements 130 between lattice cells 410-i, or may be differently configured. Fig. 4 illustrates that, in the lattice structure 440-2, the bridge elements 432 connecting the lattice cells 410-i to the bar units 450-2, 450-3 are differently shaped in that they comprise a straight section between the two sections of opposite curvature.

It should be understood that the implant 400 of Fig. 4 may be configured with any number of bar units 450-i, with any type of lattice structures 140, 240, 341, 342, 440 in between. Regarding previous variants, the bar units 450-i may replace the bar units 250-i in Fig. 2, or any or all of the short or (preferably) the long strip sections 142-i of Fig. 1B or Fig. 1C, the reason being that all of these structures may be fixed to, or aligned with, or arranged instead of, or replacing, a patient's ribs or rib remnants. Examples will follow shortly.

The entire implant 400, or any parts thereof such as the bar units 450-i and/or the lattice structures 440-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

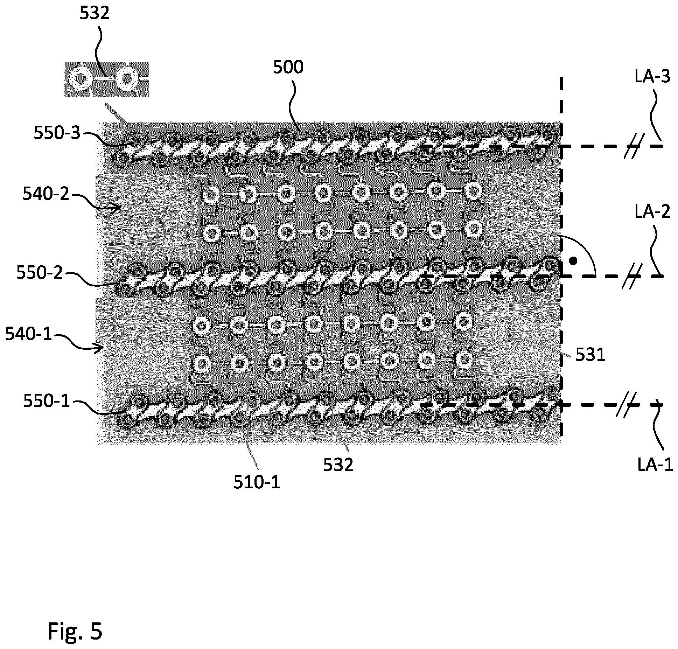

Fig. 5 illustrates an implant 500 according to yet another embodiment of the present invention. The implant 500 may be described as a variant of the implant 400 of Fig. 4, with two differences: first, the bar units 550-1, 550-2, 550-3 of the implant 500 are not only arranged in parallel but also directly aligned over one another with respect to a direction perpendicular to the longitudinal axes LA-i of the bar units 550-i.

Second, the lattice structures 540-1, 540-2 differ from those of the implant 400 in that bridge elements 532 between lattice cells 510-i (or: between ring elements 120) of the same row (parallel to the longitudinal axes LA-i) have a linear shape, i.e. the shape of thin rods. One row of such lattice cells 510-i, together with the linear (or: straight) bridge elements 532 there between could also be designated as a (smaller) bar unit. Thus, the implant 500 could also be described as consisting of bar units of two different types (one type long with two rows of through-holes, and one type short with one row of through-holes), or the lattice structures 540-i could be considered as comprising two lattice cells each, wherein the lattice cells are bar units consisting of a linear arrangement of ring elements 120 and linear bridge elements 532 there between.

By contrast, the other bridge elements 531, which connect lattice cells 510-i between different rows or between any lattice cell 510-i and any bar unit 550, may have the usual S-shape. It is evident how this limits the flexibility of the implant 500 in the direction of the longitudinal axes LA-i, while maintaining more flexibility in the direction perpendicular to them.

The entire implant 500, or any parts thereof such as the bar units 550-i and/or the lattice structures 540-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

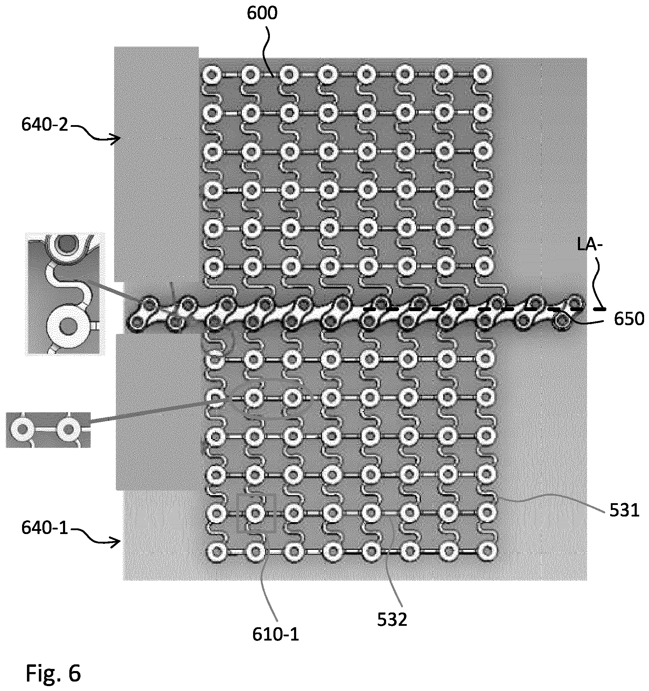

Fig. 6 illustrates an implant 600 according to still another embodiment of the present invention. The implant 600 may be seen as a variant of the implant 500 of Fig. 5, as its two lattice structures 640-1, 640-2 have the same 2-dimensional cubic body-centered lattices as the lattice structures 540-i of the implant 500. However, the implant 600 consists of only one bar unit 650, connected on each of its sides to a respective lattice structure 640-i. Bridge elements 532 which connect ring element 120 in a direction in parallel to the longitudinal axis LA of the bar unit 650 have a linear, rod-like shape. Bridge elements 531 which connect ring elements 120 in other directions (here: in directions perpendicular to the longitudinal axis LA) have the S-shape, including bridge elements 531 connecting ring elements 120 to the bar unit 650.

As in the implants 400 and 500, also in the implant 600 the length of the bar units 450-i, 550-i, 650 protrudes on both sides beyond the attached lattice structures 440-i, 540-i, 640-i, for example by one pair 451-i of through-holes, two pairs (as shown in Fig. 4-6), three pairs or more pairs. In some variants, the bar units 450-i, 550-i, 650 may protrude to different degrees beyond the lattice structures 440-i, 540-i, 640-i on the two sides, and/or different bar units 450-i, 550-i, 650 of the same implant 400, 500, 600 may protrude to different degrees and/or differently on the two sides. One advantage of the protrusion is that the bar units 450-i, 550-i, 650 may be fixed to a remaining rib section of a patient using the protruding part such that the lattice structures 440-i, 550-i, 640-i cover the bone defect.

The entire implant 600, or any parts thereof such as the bar unit 650 and/or the lattice structures 640-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

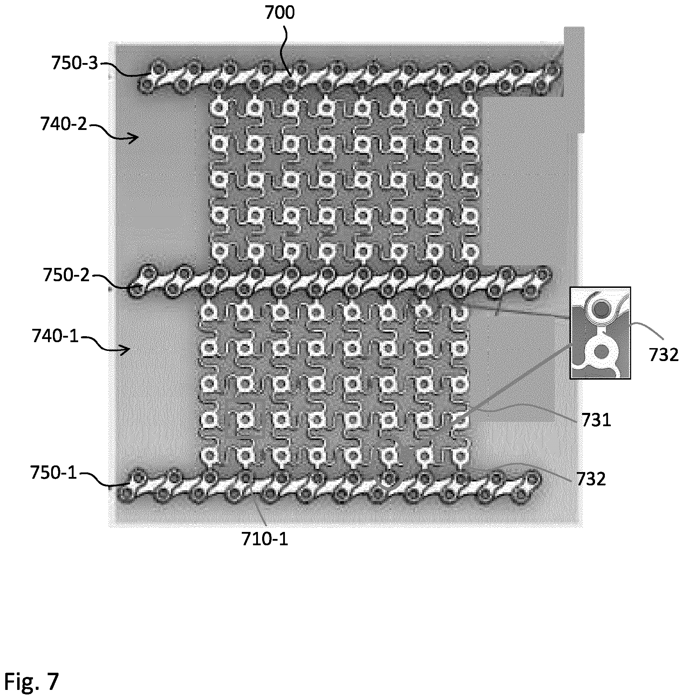

Fig. 7 illustrates an implant 700 according to yet another embodiment of the present invention. The implant 700 may be seen as a variant of the implant 400 of Fig. 4, in that it also comprises three bar sections 750-1, 750-2, 750-3, between each pair of which a corresponding lattice structure 740-1, 740-2 is sandwiched. The lattice structures 740-i are also 2-dimensional cubic body-centered lattices, wherein bridge elements 731 connecting ring elements 120 to one another are of the above described S-shape, similar to the bridge elements 431 of the implant 400.

By contrast, bridge elements 732 which connect ring elements 120 to bar units 750-i have a different shape: they are linear in shape and preferably shorter than the diameter of the ring elements 120. This adds additional stiffness between the bar units 750-i and the adjacent ring elements 120 such that stiffness gradually wanes from the bar units 750-i towards the center of the lattice structures 740-i there between. This means that, when the bar units 750-i are fixed to remnants of ribs, just as natural ribs they provide stiffness in their immediate environment, whereas the lattice structures 740-i in between offer more flexible support to the tissue in between.

The lattice structures 740-1, 740-2 of the implant 700 consist of five rows of lattice cells 710-i with eight lattice cells 701-i each, the rows being arranged in parallel to the longitudinal axes of the bar units 750-i. Expressed differently, the lattice structures 740-1, 740-2 consist of eight columns of lattice cells 710-i with five lattice cells 710-i each, wherein each column is arranged perpendicular to the longitudinal axes of the bar units 750-i and aligned with a closer one of the through-holes of a respective pair of through-holes of the bar unit 750-i on either side.

Since the two through-holes of each pair of through-holes are slanted with respect to the longitudinal axes of the bar units 750-i, this means that the columns aligned with the same pair of through-holes on both sides of the bar unit 750-i are not aligned with one another, but are slightly shifted in the longitudinal direction of the longitudinal axes. This may provide additional robustness to the implant 700 as there is no single "fault line" running perpendicular to the bar units 750-i through the entire implant 700.

The implant 700 may also be configured with a different number of rows and/or columns of lattice cells 710-i, with bar units 750-i that do not protrude on either side (or protrude differently than depicted), with more or less bar units 750-i and/or the like.

The entire implant 700, or any parts thereof such as the bar units 750-i and/or the lattice structures 740-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

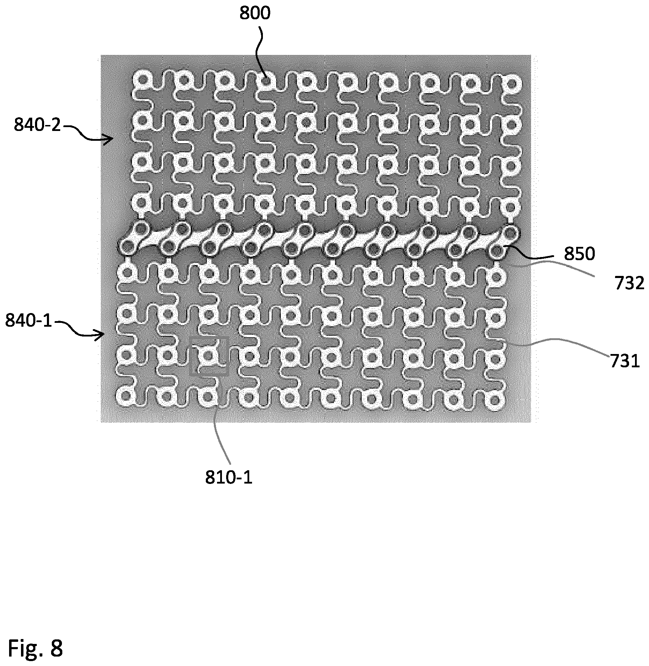

Fig. 8 illustrates an implant 800 according to yet another embodiment of the present invention. The implant 800 is a variant of the implant 700 of Fig. 7. The lattice regularity of the lattice structures 840-1, 840-2 is the same as of the lattice structure 740-1, 740-2, and also the lattice cells 810-i are configured identically to the lattice cells 710-i. The lattice cells 810-i are configured with the same types of bridges 731, 732 as the lattice cells 710-i of the implant 700, i.e., with linear bridge elements 732 between ring elements 120 and the bar unit 850, and with S-shaped bridge elements 731 between ring elements 120.

One point of contrast is that the implant 800 comprises only a single bar unit 850, which is flanked (or: sandwiched) on both sides by the two lattice structure 840-1, 840-2.

The lattice structures 840-1, 840-2 consist of four rows of lattice cells 810-i with ten lattice cells 701-i each, the rows being arranged in parallel to the longitudinal axis of the bar unit 850. Expressed differently, the lattice structures 840-1, 840-2 consist of ten columns of lattice cells 810-i with four lattice cells 7810-i each, wherein each column is arranged perpendicular to the longitudinal axis of the bar unit 850 and aligned with a closer one of the through-holes of a respective pair of through-holes of the bar unit 850-i on either side. In this variant, the bar unit 850 does not protrude from either of the two lattice structures 840-i on either side. The implant 800 may also be configured with a different number of rows and/or columns of lattice cells 810-i, with a bar unit 850 that protrudes on either side, with multiple bar units and/or the like.

The entire implant 800, or any parts thereof such as the bar units 850 and/or the lattice structures 840-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

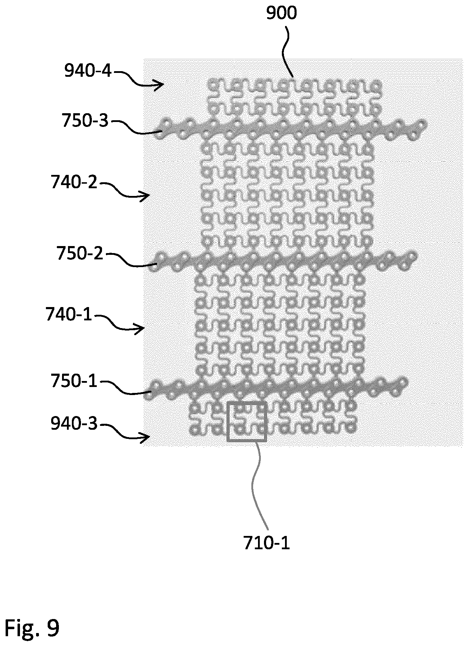

Fig. 9 illustrates an implant 900 according to yet another embodiment of the present invention. The implant 900 is a variant of the implant 700 of Fig. 7. Compared to the implant 700, the implant 900 is provided with two additional lattice structures 940-3, 940-4 such that all three bar units 750-i are sandwiched, or flanked, on both sides by lattice structures 740-i, 940-i. The additional lattice structures 940-3, 940-4 consist of the same number of columns as the lattice structures 740-i of the implant 700, but a smaller number of rows (in the shown variant specifically: two rows).

The entire implant 900, or any parts thereof such as the bar units 750-i and/or the lattice structures 740-i, 940-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

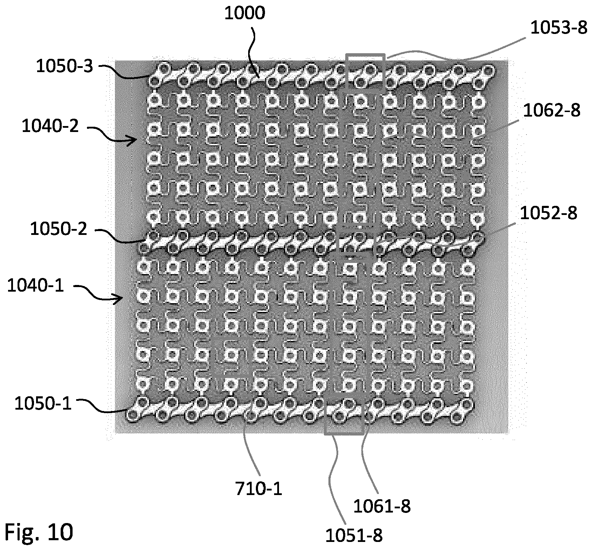

Fig. 10 illustrates an implant 1000 according to yet another embodiment of the present invention. The implant 900 is a variant of the implant 700 of Fig. 7. The difference are only two: first, in the bar units 1050-1, 1050-2, 1050-3 of the implant 1000, which, in contrast to the bar units 740-i of the implant 700, do not protrude on either side from the lattice structures 740-i. Second, in the number of lattice cells 710-i in the lattice structures 1040-1, 1040-2, which each comprise twelve columns 1061-i, 1062-i of lattice cells 710-i, each column 1061-i, 1062-i consisting of five lattice cells 710-i.

As indicated in Fig. 10, the columns 1061-i of lattice cells 710-i of the first lattice structure 740-1 are generally aligned between a corresponding pair 1051-i of through-holes of the first bar unit 1050-1 and a corresponding pair 1052-i of through-holes of the second bar unit 1050-2, or, more precisely, aligned between a closer through-hole of the corresponding pair 1051-i of through-holes of the first bar unit 1050-1 and a closer through-hole of the corresponding pair 1052-i of through-holes of the second bar unit 1050-2. Furthermore, the columns 1062-i of lattice cells 710-i of the first lattice structure 740-2 are generally aligned between a corresponding pair 1052-i of through-holes of the second bar unit 1050-2 and a corresponding pair 1053-i of through-holes of the third bar unit 1050-3, or, more precisely, aligned between a closer through-hole of the corresponding pair 1052-i of through-holes of the second bar unit 1050-1 and a closer through-hole of the corresponding pair 1053-i of through-holes of the third bar unit 1050-3. Consequently, the bar units 1050-i also comprise (or consist of) twelve pairs 1051-j, 1052-j, 1053-j of through-holes each.

In Fig. 10, the situation is illustrated by marking the eighth (from the left) pair 1051-8 of through-holes of the first bar unit 1050-1, the eighth column 1061-8 of the first lattice structure 740-1, the eighth pair 1052-8 of through-holes of the second bar unit 1050-2, the eighth column 1062-8 of the second lattice structure 740-2, and the eighth pair 1053-8 of through-holes of the third bar unit 1050-3.

The entire implant 1000, or any parts thereof such as the bar units 1050-i and/or the lattice structures 740-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

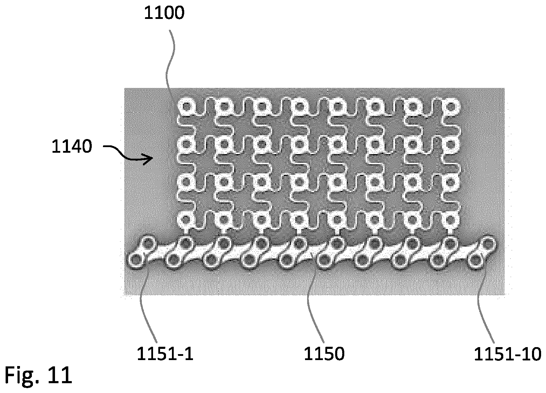

Fig. 11 illustrates an implant 1100 according to yet another embodiment of the present invention. The implant 1100 is yet another variant of the implant 700 of Fig. 7. As in the previous variants of the implant 700, the types of bridge elements and their configuration is the same as in the implant 700. The difference between implant 1100 and implant 700 is that implant 1100 consists of a single bar unit 1150 and a single lattice structure 1140 attached on one side of the bar unit 1150. The lattice structure 1140 consists of eight rows of lattice cells 710-i, each row consisting of four lattice cells 710-i. The bar unit 1150 protrudes on both ends by one pair 1051-1, 1051-10 of through-holes, respectively, and thus comprises (or consist of) ten pairs 1051-i.

The entire implant 1100, or any parts thereof such as the bar units 1150 and/or the lattice structures 1140, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

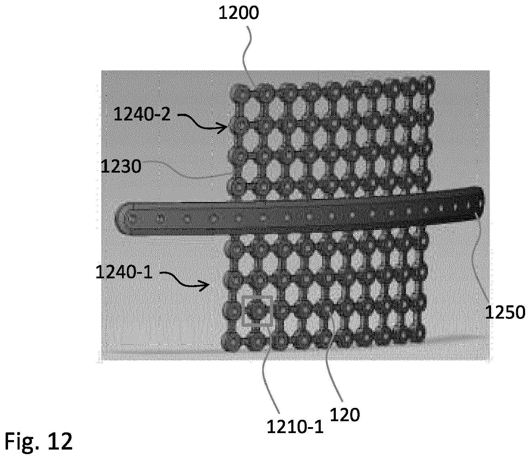

Fig. 12 illustrates an implant 1200 according to a further embodiment of the present invention. The implant 1200 consists of a single bar unit 1250 which is sandwiched on both sides by a respective lattice structure 1240-1, 1240-2. Each lattice structure 1240-i consists of ten columns of four lattice cells 1210-i each. All of the bridge elements 1230, both those between ring elements 120 as well as between ring elements 120 and bar unit 1250, have a linear shape. The bar unit 1250 comprises only a single row of regularly-spaced through-holes and is configured as a rectangular strip with rounded corners. Each of the columns of the lattice structures 1240-i is aligned with one of the through-holes of the bar unit 1250. The bar unit 1250 protrudes, on both ends by four through-holes.

The entire implant 1200, or any parts thereof such as the bar units 1250-i and/or the lattice structures 1240-i, may be made from a material comprising, or consisting of: polyetheretherketone, PEEK, polyethylen,PE, polyphenylsulfone, PPSU, polyetherketoneketone, PEKK, Magnesium, Molybdenum, and/or a compound therewith, and/or a compount including hydroxylapatite, HA, tricalciumphosphate, TCP, Silver, and/or Magnesium.

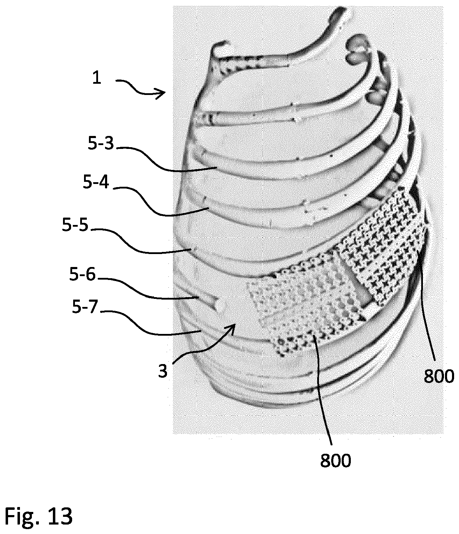

Fig. 13 illustrates a possible application of the implant 800 of Fig. 8. It shows a human thorax 1 with a large-scale bone defect 3. Specifically, the large-scale bone defect 3 consists in a large portion (over 10%) of a rib 5-6 missing. The bone defect 3 is covered by two implants 800. The implants 800 are fixed to the two neighboring ribs 5-5, 5-7 adjacent to the bone defect 3 by fixation devices being applied, through through-holes of the outermost row of lattice cells 810-i of each of the two lattice structure 840-i of each implant 800. The two implants 800 are arranged roughly in line with one another, with an angle between the longitudinal axes of the two implants given by the natural curvature of the ribs 5-5, 5-7. The single bar unit 850 of each implant 800 remains unfixed. It is evident how the two bar units 850 of the two implants 800 arranged roughly in line substitute for the missing parts of the rib 5-6 in the bone defect 3.

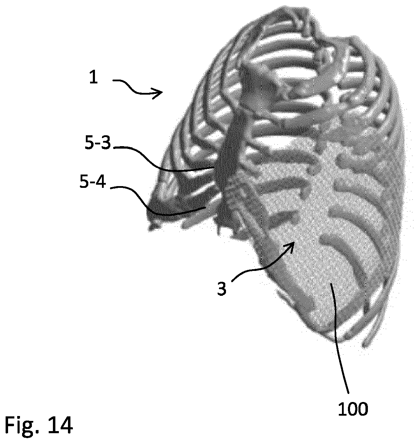

Fig. 14 illustrates a possible application of the implant 100 of Fig. 1A. The thorax 1 in Fig. 14 has an even larger bone defect 3 than the one shown in Fig. 13, the bone defect 3 comprising missing sections of several ribs 5-i, in particular of at least five ribs 5-i. The mesh-like implant 100 is fixed, using the through-holes 110-i of the ring elements 120, to several of the ribs 5-i, wherein the area in between is covered by the lattice structure 140.

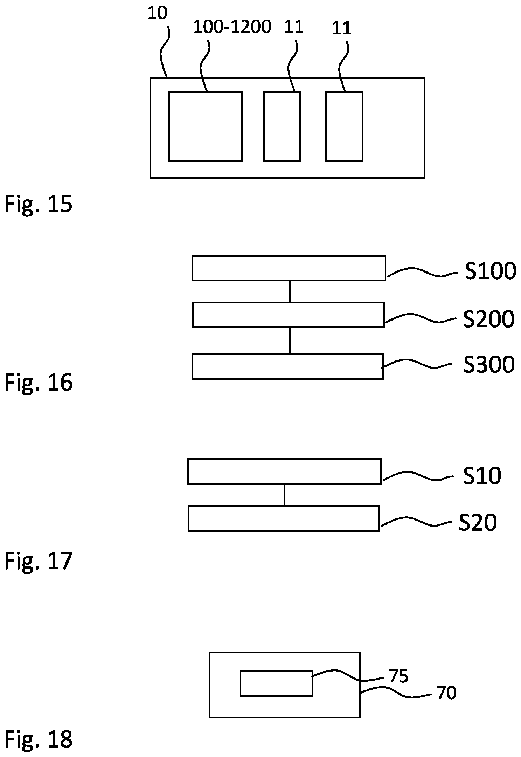

Fig. 15 shows a system 10 for covering a large-scale bone defect according to an embodiment of the second aspect of the present invention. The system 10 comprises at least one implant according to any embodiment of the first aspect of the present invention, in particular at least one of the implants 100-1200 as have been described in the foregoing. The system 10 also comprises at least one, preferably at least two, fixation devices 11, configured to be received by through-holes (or: orifices) in the implants 100-1200, for fixing the implant 100-1200 to bone, i.e. to one or more bones (complete or partial) of a patient.

A fixation device 11 can be made of a material comprising, or consisting of, Titanium, a polymer, or any other suitable material. The fixation device 11 can be, for example, a screw (e.g., a Titanium screw) or a more complex apparatus, for example comprising a sonic-weldable part. Any fixation device 11 may comprise, or consist of, bio-degradable or non-bio-degradable metal screws or pins, or resorbable or non-resorbable polymeric pins or screws.

Fig. 16 shows a flow diagram illustrating a method for covering a large-scale bone defect 3 (such as any of the bone defects in Fig. 13 or Fig. 14), in particular using any of the implants 100-1200 as shown in the foregoing.

In a step S100, a system 10 according to any embodiment of the present invention is provided, in particular a system comprising any of the implants 100-1200 that have been described in the foregoing and any suitable fixation device 11.

In a step S300, the implant 100-1200 is fixed to at least two portions of bone 5-i of a patient using the fixation devices 11. The portions of bone may specifically be portions of one or more ribs 5-i of a patient, some or all of which may have been partially resected.

The system 10 may in particular comprise the implant 800 of Fig. 8, being fixed as shown in Fig. 13. Specifically, the implant 100-1200 may comprise at least one bar unit 150-1250, wherein the implant 100-1200 is fixed, by one or more ring elements 120 of at least one lattice structure 14-1240, to at least one rib 5-i of a patient, such that the at least one bar unit 150-1250 substitutes at least a missing section of another rib 5-6 of the patient. Similarly, the implant 100-1200 may comprise at least three bar units 150-1250, wherein the implant 100-1200 is fixed with a first bar unit to, and generally along, a first rib 5-5 of a patient, and with a second bar unit to, and generally along, a second rib 5-7 of a patient, while a third bar unit arranged between the first and the second bar unit is not fixed to a portion of bone 5-6, preferably such that the third bar unit substitutes a missing portion of a rib 5-6 or an entirely missing rib of the patient by being arranged roughly (or exactly) where the missing portion or rib would be and oriented roughly (or exactly) in the same orientation (see Fig. 13).

In an optional step S200, the implant 100-1200 provided in step S100 may be specifically tailored after manufacturing (specifically after having been additively manufactured), e.g. by a surgeon, to a specific bone defect 3 and/or a specific patient by removing at least part of the implant 100-1200, e.g. using surgical scissors, a scalpel, and/or any other medical instrument. Removing a part of the implant 100-1200 may specifically include removing parts of at least one lattice structure.

Fig. 17 shows a flow diagram schematically illustrating a method according to an embodiment of the fourth aspect of the present invention, i.e. a method for producing the implant of any embodiment of the first aspect of the present invention.

The method comprises a step S10 of providing data defining a digital representation of the implant according to any embodiment of the present invention, in particular of any of the implants 100-1200 or variants thereof.

The method further comprises a step S20 of additively manufacturing (or: "3D-printing") the implant based on the digital representation thereof.

The data provided in step S10 may further comprise operating instructions adapted to control an additive manufacturing device using said digital representation thereof, which may then be used in step S20. Additive manufacturing may be performed at a printing temperature of over 180°C, and with printed layers of between 0,1mm and 0,5mm of thickness.

Preferably, the entire implant is manufactured using additive manufacturing in this way.

Alternatively, the step S20 may be a step of manufacturing the implant 100-1200 using mold injection.

Fig. 18 shows a block diagram schematically illustrating a computer-readable, non-volatile data storage medium 70 according to a fifth aspect of the present invention, i.e. a data storage medium 70 comprising data 75 defining a digital representation of the implant according to any embodiment of the first aspect of the present invention, for example of any of the implants 100-1200. Optionally, the data 75 may further comprise operating instructions configured to control an additive manufacturing device to manufacture the implant based on the digital representation of the implant.

Herein, several units or elements of the same type in one embodiment may be numbered by a reference sign such as "110" (here as an example, any other alphanumeric reference sign may be used), followed by a dash and a number referencing the individual unit or element, e.g., 110-1, 110-2 and so on. This is done to distinguish individual units/elements from one another and may sometimes be used to indicate all of the units/elements of the same type, in particular in the term "110-1, 110-2, ..., 110-8". The last element may be designated as "110-N" without prejudice. All of the elements collectively, or a random single element, may both be designated by "110-i", without loss of generality, in particular when discussing properties common to all of them. The values of "i" may change between paragraphs or sentences, depending on the context. If distinction is deemed important, other small letters (such as "j", "k", and so on) may be used in the same manner as "i", for example in phrases such as "even-numbered elements 110-i and odd-numbered elements 110-j". In other contexts, the "-i" may be simply left out, indicating the entire type of unit/element, disregarding any difference between the individual instances "-i" thereof.

The invention comprises at least the following embodiments:
Embodiment 1. An implant for covering a thorax bone defect, comprising:
   at least one lattice structure of individual lattice cells,
   each lattice cell comprising one or more ring elements, each ring element comprising a through-hole configured for receiving a fixation device,
   at least some of the ring elements of each lattice structure being connected via linear or non-linear bridge elements to one another,
   wherein the implant is made at least predominantly from a plastic material.
Embodiment 2: The implant of Embodiment 1, being made from a plastic material using additive manufacturing, AM, and/or injection molding.
Embodiment 3; The implant of Embodiment 1 or 2, wherein the implant is made from a material comprising, or consisting of: polyetheretherketone, PEEK, Polyethylen, PE, polyphenylsulfone, PPSU, polyetherketoneketone, PEKK, Magnesium, Molybdenum and/or a compound therewith.
Embodiment 4: The implant of any of Embodiments 1 to 3, wherein the implant is made from a material comprising, or consisting of: a compound including hydroxylapatite, HA, tricalciumphosphate, TCP, Silver, and/or Magnesium.
Embodiment 5: The implant of any of Embodiments 1 to 4, wherein at least the at least one lattice structure has anti-bacterial properties.
Embodiment 6: The implant of any of Embodiments 1 to 5, wherein the at least one lattice structure has a thickness of from about 0.1 mm to about 5 mm.
Embodiment 7: The implant of any of Embodiments 1 to 6, wherein the lattice structure is an essentially 2-dimensional lattice, in particular a triclinic lattice, a monoclinic lattice, an orthorhombic lattice, a tetragonal lattice, a hexagonal lattice or a cubic lattice.
Embodiment 8: The implant of any of Embodiments 1 to 7, wherein at least some lattice cells consist of a single ring element, together with at least a portion of at least one bridge element.
Embodiment 9: The implant of any of Embodiments 1 to 8, wherein all connections between all ring elements are non-linear.
Embodiment 10: The implant of any of Embodiments 1 to 9, wherein the implant comprises at least one lower-density lattice cell, LDLC, and at least one higher-density lattice cell, HDLC, having the same size and contour as the at least one lower-density lattice cell, LDLC, but comprising a higher number of ring elements.
Embodiment 11: The implant of any of Embodiments 1 to 10 wherein the implant comprises at least one lattice structure and at least one bar unit, the lattice structures and the bar units being arranged in a regular pattern.
Embodiment 12: The implant of Embodiment 11, wherein the lattice structures and the bar units are arranged alternatingly.
Embodiment 13: The implant of Embodiment 11 or 12, wherein lattice cells immediately adjacent to bar units are connected to the bar units by linear bridge elements.
Embodiment 14: The implant of any of the Embodiments 11 to 13, wherein the bridge elements connecting ring elements in a direction in parallel to the longitudinal direction of the at least one bar unit are linear bridge elements.
Embodiment 15: The implant of any of the Embodiments 11 to 13, comprising a plurality of bar units arranged in a staggered manner with respect to one another.
Embodiment 16: The implant of any of the Embodiments 11 to 15, comprising a plurality of bar units arranged in parallel with one another, and exactly aligned with respect to their longitudinal ends.
Embodiment 17: The implant of any of the Embodiments 11 to 16, wherein each bar unit comprise at least one line of through-holes for receiving fixation devices.
Embodiment 18: The implant of any of the Embodiments 11 to 17, wherein the at least one bar unit comprises a plurality of pairs of through-holes, the pairs being arranged along the longitudinal axis of the bar unit.
Embodiment 19: The implant of Embodiment 18, wherein the pairs of the plurality of pairs of through-holes are arranged in parallel to each other but neither in parallel nor perpendicular to the longitudinal axis of the at least one bar unit.
Embodiment 20: A system for covering a large-scale bone defect, comprising the implant of any of the Embodiments 1 to 19 as well as a plurality of fixation devices.
Embodiment 21: The system of Embodiment 20, wherein the fixation devices comprise, or consist of, bio-degradable or non-bio-degradable metal screws or pins, or resorbable or non-resorbable polymeric pins or screws.
Embodiment 22: A method for covering a thorax bone defect, comprising:
   providing the system of claim Embodiment 20 or 21; and
   fixing the implant to at least two portions of bone of a patient using the fixation devices.
Embodiment 23: The method of Embodiment 22, wherein the at least two portions of bone are portions of one or more ribs, wherein in particular one or more ribs have been partially resected.
Embodiment 24: The method of Embodiment 22 or Embodiment 23, wherein the system comprises an implant according to Embodiment 10 with at least one bar unit, wherein the implant is fixed, by one or more ring elements of at least one lattice structure, to at least one rib of a patient, such that the at least one bar unit substitutes at least a missing section of another rib of the patient.
Embodiment 25: The method of any of Embodiments 22 to 2, wherein the implant, after manufacturing, is tailored, particularly by removing parts of at least one lattice structure, to a specific patient and/or a specific bone defect.
Embodiment 26: A method for producing the implant of any of Embodiments 1 to 19, comprising additive manufacturing or injection molding of the entire implant.
Embodiment 27: A data structure which defines a digital representation of the implant of any of Embodiments 1 to 19.
Embodiment 28: A non-volatile, computer-readable data storage medium storing data which defines a digital representation of the implant of any of Embodiments 1 to 19.
Embodiment 29: A data stream comprising, or configured to generate, data defining a digital representation of the implant of any of Embodiments 1 to 19.
Embodiment 30: The data structure of Embodiment 27 or the data storage medium of Embodiment 28 or the data stream of Embodiment 29, the data further defining operating instructions adapted to control an additive manufacturing device to fabricate the implant using the digital representation of the implant when said data is relayed to the additive manufacturing device.

### List of Reference Signs

- 1: thorax
- 3: bone defect
- 5-i: rib
- 10: system for covering large-scale bone defects
- 11: fixation device
- 70: data storage medium
- 75: data defining digital representation
- 100: implant
- 100': implant
- 100": implant
- 110-i: lattice cell
- 120: ring element
- 130: bridge element
- 140: lattice structure
- 141-i: long strip section
- 142-i: short strip section
- 200: implant
- 210-i: lattice cell
- 240: lattice structure
- 250-i: bar unit
- 300: implant
- 311-i: lower-density lattice cell, LDLC
- 312-i: higher-density lattice cell, HDLC
- 340: lattice structure
- 341-i: first type of strip section
- 342-i: second type of strip section
- 400: implant
- 410-i: lattice cell
- 431: bridge element
- 432: bridge element
- 440-i: lattice structure
- 450-i: bar unit
- 500: implant
- 510-i: lattice cell
- 531: S-shaped bridge element
- 532: linear bridge element
- 540-i: lattice structure
- 550-i: bar unit
- 600: implant
- 610-i: lattice cell
- 640-i: lattice structure
- 650: bar unit
- 700: implant
- 710-i: lattice cell
- 731: S-shaped bridge element
- 732: linear bridge element
- 740-i: lattice structure
- 750-i: bar unit
- 800: implant
- 810-i: lattice cell
- 840-i: lattice structure
- 850: bar unit
- 900: implant
- 940-i: lattice structure
- 1000: implant
- 1050-i: bar unit
- 1051-i: pair of through-holes in the first bar unit 1050-1
- 1052-i: pair of through-holes in the second bar unit 1050-2
- 1053-i: pair of through-holes in the third bar unit 1050-3
- 1061-i: column of lattice cells in the first lattice structure 740-1
- 1062-i: column of lattice cells in the second lattice structure 740-2
- 1100: implant
- 1140: lattice structure
- 1150: bar unit
- 1151-i: pair of through-holes
- LA-i: longitudinal axis
- S10..S20: method steps
- 5100.. 5300: method steps

## Claims

1. An implant (100-1200) for covering a thorax bone defect(3), comprising:
at least one lattice structure (140-1240) of individual lattice cells (110-810),
each lattice cell (110-810) comprising one or more ring elements (120), each ring element (120) comprising a through-hole configured for receiving a fixation device (11),
at least some of the ring elements (120) of each lattice structure (140-1240) being connected via linear or non-linear bridge elements (130, 431, 432, 531, 532, 731, 732) to one another,
wherein the implant (100-1200) is made at least predominantly from a plastic material.

2. The implant (100-1200) of claim 1, being made from a plastic material using additive manufacturing, AM, and/or injection molding.

3. The implant (100-1200) of claim 1 or 2, wherein the implant is made from a material comprising, or consisting of: polyetheretherketone, PEEK, Polyethylen, PE, polyphenylsulfone, PPSU, polyetherketoneketone, PEKK, Magnesium, Molybdenum and/or a compound therewith, or a compound including hydroxylapatite, HA, tricalciumphosphate, TCP, Silver, and/or Magnesium.

4. The implant (100-1200) of any of claims 1 to 3, wherein the at least one lattice structure (140-1240) has a thickness of from about 0.1 mm to about 5 mm.

5. The implant (100-1200) of any of claims 1 to 3, wherein the lattice structure is an essentially 2-dimensional lattice, in particular a triclinic lattice, a monoclinic lattice, an orthorhombic lattice, a tetragonal lattice, a hexagonal lattice or a cubic lattice.

6. The implant (100-1200) of any of claims 1 to 5, wherein at least some lattice cells (110-810) consist of a single ring element (120), together with at least a portion of at least one bridge element (130, 431, 432, 531, 532, 731, 732).

7. The implant (300) of any of claims 1 to 6, wherein the implant (300) comprises at least one lower-density lattice cell, LDLC (311), and at least one higher-density lattice cell, HDLC (312), having the same size and contour as the at least one lower-density lattice cell, LDLC (311), but comprising a higher number of ring elements (120).

8. The implant (200; 400-1200) of any of claims 1 to 7, wherein the implant (200; 400-1200) comprises at least one lattice structure (240; 440-1240) and at least one bar unit (250; 450-1250), the lattice structures (240; 440-1140) and the bar units (250; 450-1250) being arranged in a regular pattern.

9. The implant (500; 600; 1200) of claim 8, wherein the bridge elements (532) connecting ring elements (120) in a direction in parallel to the longitudinal direction of the at least one bar unit (550; 650; 1250) are linear bridge elements (532).

10. The implant (400; 500; 700; 1000) of claim 8 or 9, comprising a plurality of bar units (450-1) arranged in a staggered manner with respect to one another, and/or a plurality of bar units (550; 750; 1050) in parallel with one another and exactly aligned with respect to their longitudinal ends.

11. A system (10) for covering a large-scale bone defect (3), comprising the implant (100-1200) of any of claims 1 to 10 as well as a plurality of fixation devices (11).

12. The system (10) of claim 11, wherein the fixation devices (11) comprise, or consist of, bio-degradable or non-bio-degradable metal screws or pins, or resorbable or non-resorbable polymeric pins or screws.

13. A method for producing the implant (100-1200) of any of claims 1 to 10, comprising additive manufacturing (S20) or injection molding of the entire implant.

14. A non-volatile, computer-readable data storage medium (70) storing data (75), which defines a digital representation of the implant (100-1200) of any of claims 1 to 10.

15. The data storage medium (70) of claim 14, the data (75) further defining operating instructions adapted to control an additive manufacturing device to fabricate the implant (100-1200) using the digital representation of the implant (100-1200) when said data is relayed to the additive manufacturing device.
